(19) Europäisches Patentamt
European Patent Office
Office européen des brevets

(11) Publication number: 0 212 933 B1

(12) EUROPEAN PATENT SPECIFICATION

(45) Date of publication of patent specification: 24.10.90

(51) Int. Cl.$^5$: A 61 L 33/00, C 08 B 37/10

(21) Application number: 86306231.1

(22) Date of filing: 12.08.86

# (54) Method of preparing antithrombogenic medical material.

(30) Priority: 12.08.85 JP 177450/85

(43) Date of publication of application:
04.03.87 Bulletin 87/10

(45) Publication of the grant of the patent:
24.10.90 Bulletin 90/43

(84) Designated Contracting States:
BE CH DE FR GB IT LI NL SE

(56) References cited:
EP-A-0 092 414

**The file contains technical information submitted after the application was filed and not included in this specification**

(73) Proprietor: KOKEN CO. LTD.
5-18 Shimoochiai 3-chome
Shinjuku-ku Tokyo (JP)

(72) Inventor: Noishiki, Yasuharu
827 Yamada, Misasa-cho
Tohaku-gun Tottori-ken (JP)
Inventor: Kodaira, Kazuhiko
3770-16, Jindaiji
Mitaka-shi Tokyo (JP)
Inventor: Furuse, Masayasu
3-8-9, Minamidai
Sagamihara-shi Kanagawa-ken (JP)
Inventor: Miyata, Teruo
3-6-29 Shimoochiai Shinjuku-ku
Tokyo (JP)

(74) Representative: Crisp, David Norman et al
D. YOUNG & CO. 10 Staple Inn
London, WC1V 7RD (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a method of preparing an antithrombogenic medical material.

Many medical materials used in the treatment of cardiovascular diseases are required to possess antithrombogenic properties. For instance, in the case of artificial blood-vessels, artificial valves, artificial hearts, and parts of artificial lung devices, if the surfaces coming into contact with blood possess blood coagulating properties serious problems such as thrombosis may arise. In order to alleviate such problems, a variety of antithrombogenic medical materials consisting of synthetic polymer materials such as a polyurethane have been developed. These materials, however, do not always possess sufficient compatibility with endothelial cells and other cells of living tissues, and therefore with these materials it is difficult to obtain satisfactory antithrombogenic properties.

Collagen is a protein which is present in connective tissue and base membranes of animal and is highly compatible with the cells. Since collagen has no antithrombogenic property itself, it is necessary to provide collagen with antithrombogenic properties if the collagen is to be used as a medical material coming into contact with blood. A simple and safe way of providing collagen with antithrombogenic properties is to combine heparin with the collagen through a protamine, in which the protamine is fixed to the collagen through a cross-linking agent. For instance, Japanese Patent Application Laid Open No. 58180162 discloses an antithrombogenic medical material comprising a heparinized collagen in which heparin is attached to a protamine which is fixed to a collagen of animal origin through glutaraldehyde as a cross-linking agent. The heparin in the heparinized collagen forms an ionic-bond with protamine and is released slowly in the living body to prevent blood from coagulating. It is further noted that since endothelial cells grow on the collagen base, antithrombogenic properties are maintained by the endothelial cells after the heparin is entirely released.

Since, however, glutaraldehyde is used as the crosslinking agent for fixing protamine to the collagen, the resulting heparinized collagen has reduced flexibility and forms constrictions or cracks when it is bent to a small radius of curvature. It also seems that the glutaraldehyde discolors the heparinized collagen to a brown colour, and that the glutaraldehyde is polymerized and released slowly as a polymer under some conditions in the living body, which may cause toxicity over long term use.

We have now found it possible to provide a method of preparing an antithrombogenic medical material that causes no toxicity and discoloration, and possesses improved histocampatibility and antithrombogenic properties as well as improved physical characteristics, particularly, flexibility.

Thus, according to one aspect, the invention provides a method of preparing an antithrombogenic medical material comprising a heparinized collagen as an antithrombogenic component, which comprises the steps of fixing a protamine to a collagen or derivative thereof through a polyepoxy compound, and heparinizing the collagen by fixing heparin to the protamine.

Collagens and derivatives thereof which may be used in the present invention include, for example, insoluble collagens; soluble collagens; atelocollagens prepared by removing telopeptides from the ends of the collagen molecule using a protease other than collagenase; chemically modified collagens obtained by succinylation or esterification of above-described collagens; collagen derivatives such as gelatin; polypeptides obtained by hydrolysis of collagen; and natural collagens present in natural tissues (such as ureters, blood-vessels, pericardium, etc.).

Protamines are basic nucleoproteins and can be collected and purified from any animal, and may contain histones. Protamines in the form of a salt-like combination with an inorganic salt or an organic salt are preferred, and, in particular, protamine sulfate or protamine hydrochloride are preferred.

Polyepoxy compounds which may be used in the present invention include, for example, glycol diglycidyl ether, polyol polyglycidyl ether, dicarboxylic acid diglycidylester and so on. A polyethylene glycol diglycidyl ether represented by the following formula (I):

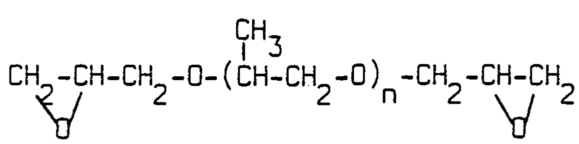
... (I)

(in which n is an integer) is particularly preferred because it can give the heparinized collagen both flexibility and hydrophilic properties.

Antithrombogenic medical materials prepared by the method according to the present invention may if desired be composites of heparinized collagens and synthetic polymer materials. The synthetic polymer materials may be products prepared by weaving or knitting polyester fibers in the form of a tube, a plastic (for example, polycarbonate) box part as in the circuit of an artificial lung device, and so on.

Preferred techniques for the preparation of heparinized collagens in accordance with the present invention are described below.

When starting from an insoluble collagen, the insoluble collagen is conveniently immersed in a 0.1% to 20% aueous protamine and subsequently in a 0.1% to 30%, preferably 1% to 10%, aqueous solution of polyepoxy compound to fix the protamine to the collagen. The protamine-fixed collagen is then subjected

to heparinization by immersing it in 0.01% to 10%, preferably 0.1% to 2% aqueous heparin. In the above immersion treatments the temperature may be in the range from 5°C to 90°C, preferably from 10°C to 35°C, and the duration of immersion may be in the range of from 10 minutes to 24 hours, preferably from 30 minutes to 8 hours.

In an alternative technique, a soluble collagen is used as the starting collagen and a heparinized collagen is obtained by the process in which a substrate such as a synthetic polymer material and the like is subjected to a coating or impregnating treatment with a solution containing the soluble collagen and a protamine, and the substrate is subsequently immersed in an aqueous solution of a polyepoxy compound, and further immersed in a heparin aqueous solution.

In another method, a heparinized collagen is obtained by a process in which a substrate such as a synthetic polymer material is subjected to a coating or impregnating treatment with a solution containing a soluble collagen, a protamine and a polyepoxy compound, and the substrate so treated is further immersed in an aqueous heparin.

In a further method, a heparinized collagen is obtained by a process in which a substrate such as a synthetic polymer material is subjected to a coating or impregnating treatment with a solution containing a soluble collagen, a protamine, and heparin, and the substrate so treated is further immersed in an aqueous solution of a polyepoxy compound.

In a still further technique, a heparinized collagen is obtained by the process in which a substrate such as a synthetic polymer material is subjected to a coating or impregnating treatment with a solution containing a soluble collagen, a protamine, a polyepoxy compound and heparin and the substrate so treated is allowed to stand until the protamine is fixed to the collagen through the polyepoxy compound and the substrate is subsequently dried.

In the foregoing exemplary processes, an insoluble collagen can be used in the form of a dispersion instead of the soluble collagen.

In the above description of heparinization, the solutions are implicitly aqueous solutions where the solvent consists only of water. It is to be noted, however, that aqueous solutions containing inorganic salts or organic substances, organic solvents or mixtures of these solvents may be used.

If desired, a mucopolysaccharide such as hyaluronic acid, chondroitin sulfate, or dermatan sulfate, may be added to any of the solutions in which collagen is present, which solutions are used in the coating or impregnating treatment of a substrate such as a synthetic polymer material, whereby the resulting heparinized collagen may be provided with increased histocompatibility and improved hydrophilic properties.

According to the present invention, it is possible to prepare an antithrombogenic medical material comprising a heparinized collagen having both superior antithrombogenic properties and high flexibility.

The following non-limiting Examples serve to illustrate the invention. In the examples, the polyethylene glycol diglycidyl ether used is Denocol EX-861 (Tradename of Nagase Sangyo K.K.) ($n \fallingdotseq 22$ in the above formula (I)).

## Example 1

The carotid (inner diameter = 3 mm; length = 10 cm) of an adult dog was immersed in 0.01% aqueous ficin (pH 7.4) at 25°C for 24 hours to remove proteins other than collagen, and the carotid was then washed well with water. With one end of the carotid closed, the hollow portion of the carotid was filled with 10% aqueous protamine sulfate (pH 5.0) and the carotid was allowed to stand at room temperature for one hour while air pressure of 100 mmHg was applied thereto. After excess solution was removed, the carotid was filled with 10% aqueous polyethylene glycol diglycidyl ether (pH 8.0) and allowed to stand for one hour in the manner described above. Then excess solution was removed. After the above treatments were repeated, the carotid was washed well with water and then immersed in 1% aqueous heparin (pH 6.0) at room temperature for one hour. The carotid, after being washed with water, was stored in 70% aqueous ethanol to give an artificial blood-vessel. No cracks and constrictions were observed in the artificial blood-vessel when it was bent by hand to a small radius of curvature.

## Example 2

A tube (inner diameter = 3 mm; length = 10 cm) for an artificial blood-vessel was prepared by knitting polyester fibers. One end of the tube was closed with a stopper, and the inside of the tube was filled with a mixture of 10 ml of 1% aqueous atelocollagen (pH 3), 5 ml of 10% aqueous protamine sulfate (pH 5) and 3 ml of 0.1% aqueous hyaluronic acid (pH 7). The tube was impregnated with the mixture by applying air pressure at 100 mmHg. After excess solution was removed, 0.1N aqueous sodium hydroxide containing 10% polyethylene glycol diglycidyl ether and 10% NaCl was poured into the tube, and the tube was allowed to stand at room temperature for two hours. The tube was washed well with water and was immersed in 1% aqueous heparin for one hour, followed by washing well with water. The tube was then immersed in 5% aqueous glycerin for two hours and freeze-dried to provide an artificial blood-vessel. The resulting artificial blood-vessel was found to have the same satisfactory flexibility as obtained in Example 1.

The artificial blood-vessels prepared in Examples 1 and 2 were implanted into the femoral aorta of an adult dog, and no thrombus was observed at all up to about three months thereafter, showing a 100% open rate.

Example 3

1 g of protamine sulfate, 0.1 g of heparin, and 10 g of reagent-grade gelatin were dissolved in 90 g of water at 50°C. The resulting solution was coated onto the inner surface of an artificial lung, and 0.1N aqueous sodium hydroxide containing 10% polyethylene glycol diglycidyl ether and 10% NaCl was poured into the artificial lung, which was then allowed to stand at 50°C for two hours, washed well with water, and then air-dried.

In a device incorporating the resulting artficial lung, no thrombus formation was observed at all for about 5 hours, showing superior antithrombogenic properties compared to those of conventional artificial lungs.

## Claims

1. A method of preparing an antithrombogenic medical material comprising heparinized collagen as an antithrombogenic component, which comprises the steps of bonding a protamine to a collagen with a polyepoxy compound, and heparinizing said collagen by bonding heparin to said protamine.

2. A method according to claim 1, wherein the protamine is at least one of protamine sulfate and protamine hydrochloride.

3. A method according to either of claims 1 and 2 wherein the polyepoxy compound is at least one glycol diglycidyl ether, polyol poly-glycidyl ether or dicarboxylic acid diglycidyl ester.

4. A method according to claim 3, wherein the polyepoxy compound is polyethylene glycol diglycidyl ether.

5. A method according to any one of the preceding claims, wherein the antithrombogenic medical material is a composite of a heparinized collagen and a synthetic polymer.

6. A method according to claim 5, wherein the synthetic polymer material is selected from products prepared by weaving or knitting polyester fibers into the form of a tube, and plastic box parts.

7. A method according to any one of the preceding claims, wherein a substrate is subjected to a coating or impregnating treatment with a solution which contains a collagen and a protamine, and said substrate is subsequently immersed in an aqueous solution of a polyepoxy compound, and then immersed in aqueous heparin.

8. A method according to any one of claims 1 to 6, wherein a substrate is subjected to a coating or impregnating treatment with a solution containing a collagen, a protamine and a polyepoxy compound, and said substrate is further immersed in aqueous heparin.

9. A method according to any one of claims 1 to 6, wherein a substrate is subjected to a coating or impregnating treatment with a solution containing a collagen, a protamine and heparin, and said substrate is further immersed in an aqueous solution of a polyepoxy compound.

10. A method according to any one of claims 1 to 6, wherein a substrate is subjected to a coating or impregnating treatment with a solution containing a collagen, a protamine, a polyepoxy compound and heparin, and said substrate is allowed to stand until the bonding of the protamine to the collagen through the polyepoxy compound is completed, and the substrate is subsequently dried.

11. A method according to any one of claims 7 to 10, wherein at least one of hyaluronic acid, chondroitin sulfate and dermatan sulfate is added to a solution containing at least a collagen.

12. An antithrombogenic medical material comprising a heparinized collagen in which a protamine is bonded to the collagen via a polyepoxy compound, and heparin is bonded to the protamine.

13. An antithrombogenic medical material according to claim 12 wherein the heparinized collagen is in association with a synthetic substrate.

## Patentansprüche

1. Verfahren zur Herstellung eines antithrombogenen medizinischen Materials, enthaltend heparinisiertes Kollagen als antithrombogenen Bestandteil, welches die Schritte des Bindens eines Protamins mit einer Polyepoxidverbindung an ein Kollagen und Heparinisierens des Kollagens durch Bindung von Heparin an das Protamin umfaßt.

2. Verfahren nach Anspruch 1, wobei man als Protamin zumindest eine Verbindung der Gruppe Protaminsulfat und Protaminhydrochlorid verwendet.

3. Verfahren nach einem der Ansprüche 1 und 2, wobei man als Polyepoxidverbindung zumindest eine Verbindung der Gruppe Glykoldiglycidylether, Polyolpolyglycidylether oder Dicarbonsäurediglycidylester verwendet.

4. Verfahren nach Anspruch 3, wobei man als Polyepoxidverbindung Polyethylenglykoldiglycidylether verwendet.

5. Verfahren nach einem der vorangegangenen Ansprüche, wobei man als antithrombogenes medizinisches Material einen Verbundwerkstoff aus einem heparinisierten Kollagen und einem synthetischen Polymer verwendet.

6. Verfahren nach Anspruch 5, wobei man als synthetisches Polymermaterial Produkte wählt, die durch Verweben oder Verwirken von Polyesterfasern in die Form einer Röhre und von Teilen von Kunststoffbehältern hergestellt werden.

7. Verfahren nach einem der vorangegangenen Ansprüche, wobei man ein Substrat einer Beschichtungs- oder Imprägnierungsbehandlung mit einer Lösung, die ein Kollagen und ein Protamin enthält, unterwirft und das Substrat dann in eine wäßrige Lösung aus einer Polyepoxidverbindung und anschließend in wäßriges Heparin taucht.

8. Verfahren nach einem der Ansprüche 1 bis 6, wobei man ein Substrat einer Beschichtungs- oder Imprägnierungsbehandlung mit einer Lösung, die ein Kollagen, ein Protamin und eine Polyepoxidverbindung enthält, unterwirft und das Substrat weiterhin in wäßriges Heparin taucht.

9. Verfahren nach einem der Ansprüche 1 bis 6, wobei man ein Substrat einer Beschichtungs- oder Imprägnierungsbehandlung mit einer Lösung, die ein Kollagen, ein Protamin und Heparin enthält, unterwirft und das Substrat weiterhin in eine wäßrige Lösung aus einer Epoxidverbindung taucht.

10. Verfahren nach einem der Ansprüche 1 bis 6, wobei man ein Substrat einer Beschichtungs- oder Imprägnierungsbehandlung mit einer Lösung, die ein Kollagen, ein Protamin, eine Polyepoxidverbindung und Heparin enthält, unterwirft und das Substrat bis zur Beendigung der Bindung des Protamins an das Kollagen über die Polyepoxidverbindung stehen läßt und das Substrat anschließend trocknet.

11. Verfahren nach einem der Ansprüche 7 bis 10, wobei man zumindest eine Verbindung der Gruppe Hyaluronsäure, Chondroitinsulfat und Dermatansulfat zu einer Lösung, die zumindest ein Kollagen enthält, hinzufügt.

12. Antithrombogenes medizinisches Material, enthaltend ein heparinisiertes Kollagen, worin ein Protamin über eine Polyepoxidverbindung an das Kollagen und das Heparin an das Protamin gebunden sind.

13. Antithrombogenes medizinisches Material nach Anspruch 12, worin das heparinisierte Kollagen mit einem synthetischen Substrat verbunden ist.

**Revendications**

1. Procédé pour préparer un matériau antithrombogène à usage médical comprenant du collagène hépariné comme composant antithrombogène, qui comprend les étapes d'union d'une protamine à un collagène avec un composé polyépoxy et d'héparination dudit collagène par liaison d'héparine à ladite protamine.

2. Procédé selon la revendication 1, dans lequel la protamine est au moins un du sulfate de protamine et du chlorhydrate de protamine.

3. Procédé selon l'une des revendications 1 ou 2, dans lequel le composé polyépoxy est au moins un d'un éther diglycidylique de glycol, d'un éther polyglycidylique de polyol ou d'un ester diglycidylique d'acide dicarboxylique.

4. Procédé selon la revendication 3, dans lequel le composé polyépoxy est un éther diglycidylique de polyéthylèneglycol.

5. Procédé selon l'une quelconque des revendications précédentes, dans lequel le matériau antithrombogène à usage médical est un composite d'un collagène hépariné et d'un matériau polymère synthétique.

6. Procédé selon la revendication 5, dans lequel le matériau polymère synthétique est choisi parmi les produits préparés par tissage ou tricotage de fibres de polyester sous forme d'un tube et des boîtes en matière plastique.

7. Procédé selon l'une quelconque des revendications précédentes, dans lequel on soumet un substrat à un traitement d'enrobage ou d'imprégnation avec une solution qui contient un collagène et une protamine, puis on plonge ledit substrat dans une solution aqueuse d'un composé polyépoxy, puis on le plonge dans de l'héparine aqueuse.

8. Procédé selon l'une quelconque des revendications 1 à 6, dans lequel on soumet un substrat à un traitement d'enrobage ou d'imprégnation avec une solution contenant un collagène, une protamine et un composé polyépoxy et on plonge de plus ledit substrat dans de l'héparine aqueuse.

9. Procédé selon l'une quelconque des revendications 1 à 6, dans lequel on soumet un substrat à un traitement d'enrobage ou d'imprégnation avec une solution contenant un collagène, une protamine et de l'héparine et on plonge de plus ledit substrat dans une solution aqueuse d'un composé polyépoxy.

10. Procédé selon l'une quelconque des revendications 1 à 6, dans lequel on soumet un substrat à un traitement d'enrobage ou d'imprégnation avec une solution contenant un collagène, une protamine, un composé polyépoxy et de l'héparine et on laisse ledit substrat reposer jusqu'à ce que la fixation de la protamine au collagène via le composé polyépoxy soit achevée, puis on sèche le substrat.

11. Procédé selon l'une quelconque des revendications 7 à 10, dans lequel on ajoute à la solution contenant au moins un collagène au moins un de l'acide hyaluronique, du chondroïtine-sulfate et du dermatane-sulfate.

12. Matériau antithrombogène à usage médical comprenant un collagène hépariné dans lequel une protamine est fixée au collagène via un composé polyépoxy et de l'héparine est fixée à la protamine.

13. Matériau antithrombogène à usage médical selon la revendication 12, dans lequel le collagène hépariné est en association avec un substrat synthétique.